# EUROPEAN PATENT SPECIFICATION

(11) **EP 4 364 832 B1**
(45) Date of publication and mention of the grant of the patent: **22.04.2026**
(21) Application number: 23204572.4
(22) Date of filing: 19.10.2023
(51) Int. Cl.: B01F 21/20, B01F 23/41, B01F 25/433, B01F 33/30, B01F 33/81, B01F 33/82, B01F 25/00, B01F 101/21

(54) **MICROFLUIDIC CHIP AND COSMETIC MANUFACTURING APPARATUS INCLUDING SAME**
MIKROFLUIDISCHER CHIP UND VORRICHTUNG ZUR HERSTELLUNG VON KOSMETIKA DAMIT
PUCE MICROFLUIDIQUE ET APPAREIL DE FABRICATION DE PRODUIT COSMÉTIQUE LA COMPRENANT

(30) Priority: 03.11.2022 KR 20220145246
(43) Date of publication of application: 08.05.2024
(73) Proprietor: Amorepacific Corporation, Seoul 04386 (KR)
(72) Inventor: HAN, Kyungsup, 17074 Yongin-si, Gyeonggi-do, (KR); OH, Soojung, 17074 Yongin-si, Gyeonggi-do, (KR); BAEK, Heungsoo, 17074 Yongin-si, Gyeonggi-do, (KR); LEE, Jeongin, 17074 Yongin-si, Gyeonggi-do, (KR)
(74) Representative: Viering, Jentschura & Partner mbB Patent- und Rechtsanwälte

(56) References cited:
- EP-A1- 1 944 612
- US-A- 5 140 161
- US-A1- 2005 220 668
- US-A1- 2020 205 545
- US-A1- 2022 072 535

## Description

### TECHNICAL FIELD

This disclosure relates to a microfluidic chip and cosmetic manufacturing apparatus including same.

### BACKGROUND

Generally, cosmetics are used for beauty treatment, skin health, and various apparatuses are being suggested to produce cosmetics. In particular, the market for customized cosmetics reflecting the user's skin condition and user needs is expanding, and various apparatuses are being suggested to produce such customized cosmetics.

Further, various attempts are being made to produce customized cosmetics instantly to provide fresh cosmetics for the consumers.

Conventional instant cosmetic manufacturing apparatus were mostly apparatuses disposing almost finished cosmetics into cartridges and simply mixing or discharging the formulation as it is. Therefore, in order to provide various cosmetics, a large number of cartridges, or complex components (e.g., a component for stirring, a component for emulsification) become necessary. However, when such components were included, the apparatus became bigger in size, or the production cost increased due to many components.

US 5 140 161 A discloses a microfluidic chip according to the preamble of claim 1. It discloses a method for determining an analyte in a blood sample, which comprises: employing a device comprising a capillary passageway which provides the motive force for moving blood into the device, wherein said passageway contains a reagent that interacts with said blood to cause a change in the fluidity of said blood, thereby providing a detectible signal; introducing blood into said capillary passageway; allowing said blood to transit at least a portion of said passageway and react with said reagent; and detecting a change in the fluidity of said blood as a measure of the presence of an analyte in or property of said blood sample.

US 2020/205545 A1 discloses an apparatus for manufacturing cosmetic using instantaneous emulsification, the apparatus comprising: a housing which forms an outer appearance; an internal phase container which is replaceably coupled to the housing, and which stores internal phase fluid; an external phase container which is replaceably coupled to the housing, and which stores external phase fluid; a channel unit which generates emulsion by mixing the internal phase fluid provided from the internal phase container and the external phase fluid provided from the external phase container; and an operative unit which provides external force required to form and discharge emulsion at the channel unit by manipulation of a user, wherein the internal phase container and the external phase container have a pumping part which is operated by action of the operative unit, and wherein the operative unit presses the pumping part of the internal phase container and the pumping part of the external phase container at the same time by external force to discharge the internal phase fluid stored in the internal phase container and the external phase fluid stored in the external phase container to the channel unit.

### SUMMARY

The embodiments of the present disclosure are proposed to address the aforementioned problems and intend to provide a microfluidic chip and a cosmetic manufacturing apparatus including same configured to instantly produce various cosmetic contents by selecting a microfluidic chip pre-loaded with a modular material of a user's choice.

Further, it is intended to provide a microfluidic chip comprising a micro flow path configured to readily generate an emulsion by mixing and emulsifying a plurality of fluids.

Further, it is intended to provide a miniaturized cosmetic manufacturing apparatus which can be used in typical houses.

Further, it is intended to provide a cosmetic manufacturing apparatus configured to provide various cosmetic contents according to the user's needs by replaceably providing a microfluidic chip.

The present invention provides a microfluidic chip according to claim 1 and a cosmetic manufacturing apparatus according to claim 11. Further embodiments of the present invention are disclosed in the dependent claims.

The microfluidic chip and cosmetic manufacturing apparatus including same may advantageously produce various cosmetic contents instantly by selecting a microfluidic chip pre-loaded with a modular material of a user's choice

Further, the microfluidic chip and cosmetic manufacturing apparatus including same have an advantage in readily generating an emulsion by mixing and emulsifying a plurality of fluids.

Further, the microfluidic chip and cosmetic manufacturing apparatus including same have an advantage in providing a miniaturized structure which can be used in general homes.

Besides, the microfluidic chip and cosmetic manufacturing apparatus including same can advantageously provide various cosmetic contents according to the user's needs by replaceably providing a microfluidic chip.

### BRIEF DESCRIPTION OF DRAWINGS

FIG. 1 schematically shows a perspective view of a cosmetic manufacturing apparatus according to an embodiment of the present disclosure configured to be used by inserting a microfluidic chip;
FIG. 2 shows a cross-sectional view of the microfluidic chip inserted into the cosmetic manufacturing apparatus of FIG. 1;
FIG. 3 shows an enlarged view of the inlet portion, dissolution portion, and a confluence of the microfluidic chip of FIG. 2;
(a) of FIG. 4 shows a schematic view of the point at which the first fluid had started flowing in the F-F' cross section of FIG.3, and (b) of FIG. 4 shows a schematic view of the point at which a certain period of time had passed after the first fluid had flown in the F-F' cross section of FIG. 3;
FIG. 5 shows an enlarged view of the stir portion of FIG. 2;
FIG. 6 conceptually shows a flux occurring in a fluid in the stir portion of FIG.5;
FIG. 7 shows an enlarged view of the vortexing portion of FIG. 2;
FIG. 8 conceptually shows the emulsion particles formed by the vortex occurring from the vortexing portion of FIG. 7.

### DETAILED DESCRIPTION OF EXEMPLARY EMBODIMENTS

Hereinafter, specific exemplary embodiments of the present disclosure will be described in detail with reference to the accompanying drawings. Additionally, it is noted that in the description of the disclosure, the detailed description for known related configurations or functions may be omitted when it is deemed that such description may obscure essential points of the disclosure.

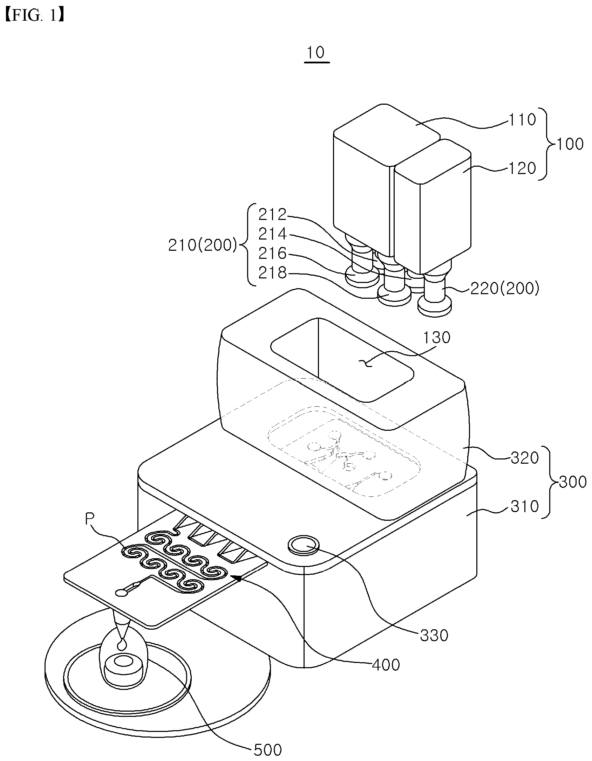

FIG. 1 schematically shows a perspective view of a cosmetic manufacturing apparatus 10 configured to be used by inserting a microfluidic chip 400 according to an embodiment of the present disclosure.

When referring to FIG. 1, the cosmetic manufacturing apparatus 10 may comprise: a storage portion 100 configured to store a plurality of fluids; a microfluidic chip 400 configured to receive the plurality of fluids stored in the storage portion 100 and form an emulsion by mixing and emulsifying with modular raw materials D1, D2 stored inside; and a main body 300 configured to accommodate the storage portion 100 and the microfluidic chip 400.

The plurality of fluids stored in the storage portion 100 flows along a path formed by the microfluidic chip 400 and dissolves the modular raw materials D1, D2, and the plurality of fluids and the dissolved modular raw materials D1, D2 are mixed in the microfluidic chip 400, formed into cosmetic contents and discharged out of the microfluidic chip 400.

The cosmetic manufacturing apparatus 10 of the present embodiment may generate various cosmetic contents (e.g., an emulsion) according to types of the microfluidic chip 400.

In the present embodiment, one kind of the microfluidic chip 400, in which the first and second modular raw materials D1, D2 are disposed on a micro flow path, will be described by way of example.

The microfluidic chip 400 is replaceably provided on the cosmetic manufacturing apparatus 10. For example, the microfluidic chip 400 is provided to be separately sold so different cosmetic contents (e.g., an emulsion) may be formed by interchanging the microfluidic chip 400.

In order to be able to generate various types of cosmetic contents according to users' preferences (e.g., an emulsion) , modular raw materials D1, D2 with various components may be stored in the microfluidic chip 400.

The cosmetic content generated in the present embodiment may comprise: an emulsion in which the plurality of fluids (e.g., first fluid and second fluid) and the modular raw materials D1, D2 are mixed and emulsified; a lotion in which the plurality of fluids and the modular raw materials D1, D2 are solubilized; and a mixed water in which the plurality of fluids and the modular raw materials D1, D2 are simply mixed.

The lotion solubilized in the present embodiment may comprise a skin lotion, an essence, and a perfume.

In this embodiment, the cosmetic content discharged from the microfluidic chip 400 is an emulsion, will be described by way of example.

In this embodiment, the cosmetic content discharged from the microfluidic chip 400 may comprise: an O/W (Oil in water) emulsion produced by evenly dispersing a hydrophobic fluid such as oil in small particle state inside a hydrophilic fluid such as water; or a W/O (Water in oil) emulsion produced by evenly dispersing a hydrophilic fluid in small particle state inside a hydrophobic fluid.

Prior to descriptions of the storage portion 100, coupling portion 200, and main body 300 forming the cosmetic manufacturing apparatus 10, the microfluidic chip 400 configured to be used by inserting into the cosmetic manufacturing apparatus 10 will be described in detail.

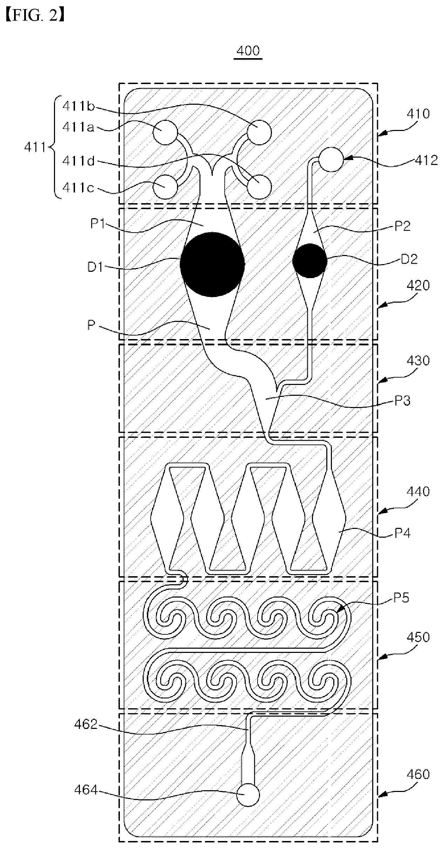

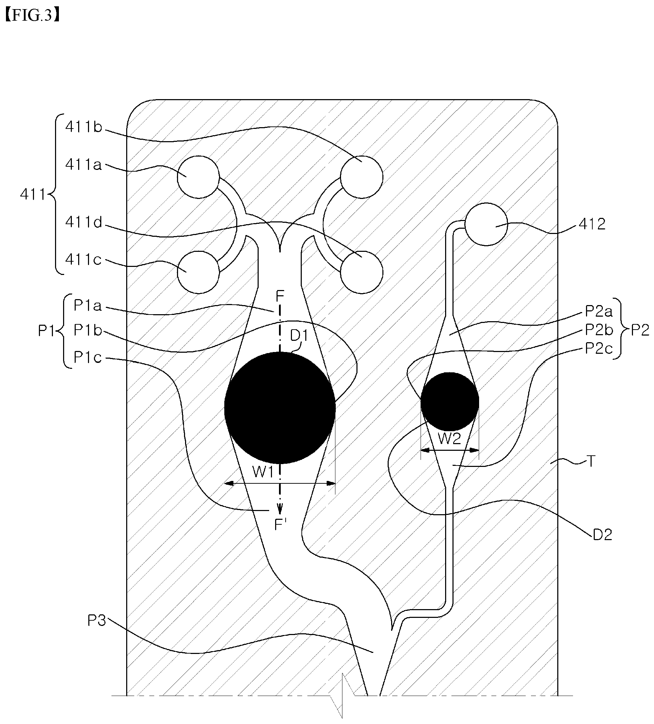

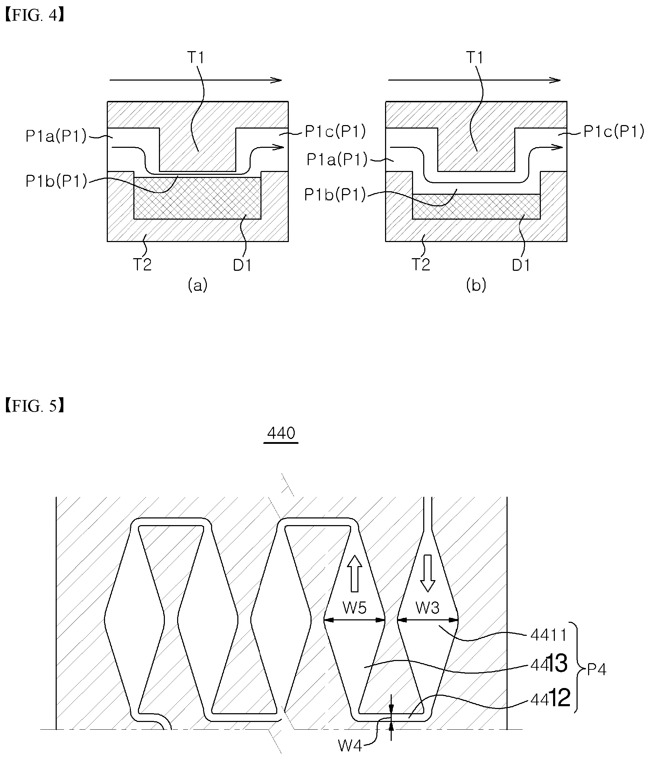

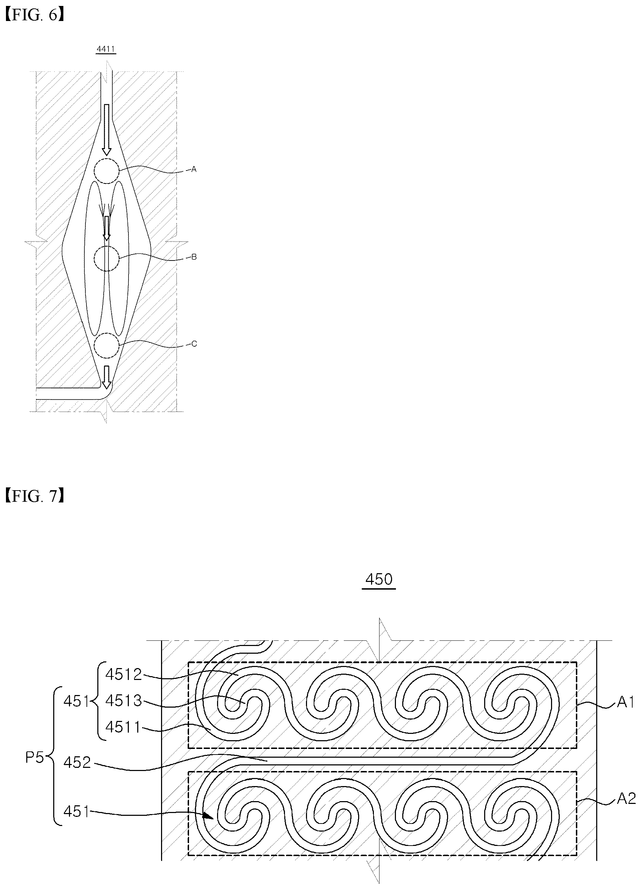

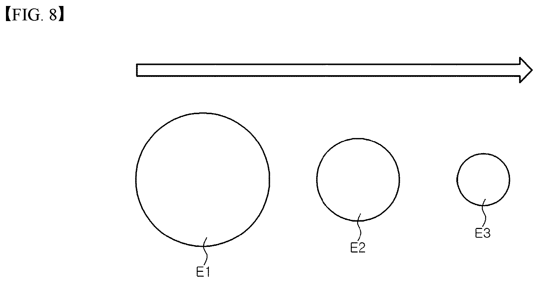

FIG. 2 shows a cross-sectional view of the microfluidic chip 400 inserted into the cosmetic manufacturing apparatus 10 of FIG. 1, FIG. 3 shows an enlarged view of an inlet portion 410, a dissolution portion 420, and a confluence 430 of the microfluidic chip 400 of FIG. 2, (a) of FIG. 4 shows a schematic view of a point at which the first fluid started flowing in F-F' cross section of FIG. 3, (b) of FIG. 4 shows a schematic view of a point at which a period of time has passed after the first fluid had started flowing in F-F' cross section of FIG. 3, FIG. 5 shows an enlarged view of a stir portion 440 of FIG. 2, FIG. 6 conceptually shows a flow of the liquid occurring in the stir portion 440 of FIG. 5, FIG. 7 is an enlarged view of a vortexing portion 450 of FIG. 2, and FIG. 8 shows an emulsion particle formed by a vortex occurring in the vortexing portion 450 of FIG. 7.

When referring to FIGS. 2 to 8, the microfluidic chip 400 according to an embodiment of the present disclosure, may comprise: the inlet portion 410 comprising a first fluid inlet 411 in which the first fluid is inserted and a second fluid inlet 412 inlet in which the second fluid is inserted; the dissolution portion 420 comprising a first micro flow path P1 in which the modular raw material D1 dissolved by the first fluid is stored and a second micro flow path P2 in which the modular raw material D2 dissolved by the second fluid is stored; the confluence 430 comprising a third micro flow path P3 in which the first fluid, the first modular raw material D1 dissolved in the first fluid, the second fluid, and the second modular raw material D2 converge; the stir portion 440 comprising a fourth fourth micro flow path P4 forming a mixed fluid by mixing the first modular raw material D1 dissolved in the first fluid, the second fluid, and the second modular raw material D2; and the vortexing portion 450 comprising a fifth micro flow path P5 configured to form a cosmetic content comprising particles by cutting the mixed fluid by creating a vortex.

The microfluidic chip 400 may be a plate with a micro flow path formed inside which a fluid can flow.

The microfluidic chip 400 may comprise various types of the second modular raw materials D2 to generate cosmetic contents (e.g., cosmetics) with different effects.

For example, the storage portion 100 may be provided in a plurality to be interchanged on the cosmetic manufacturing apparatus 10, and each of the modular raw materials (modular raw materials D1, D2) pre-loaded in the microfluidic chip 400 may be provided to have components different from each other.

In the present embodiment, two types of fluids (the first fluid, the second fluid) and the two types of modular raw materials D1, D2 dissolved by them are provided, will be described by way of example. However, the technical idea of the present embodiment is not limited to such, and that more than two types of fluids and two types of modular raw materials are provided may be comprised.

Types of the microfluidic chip 400 can be differentiated by the component and the quantity of the second modular raw material D2 pre-loaded in the microfluidic chip 400.

The microfluidic chip 400 may be provided to have different modular raw materials D1, D2 to be able to generate cosmetic contents (e.g., emulsion) with different effects.

For example, the microfluidic chip 400 is replaceably provided in a plurality on the main body 300, and the modular raw material (e.g., modular raw materials D1, D2) disposed in the plurality of microfluidic chips 400 may be provided to have components different from each other.

Further, the micro flow paths P formed on the plurality of microfluidic chips 400 may be formed to have structures different from each other depending on the disposed modular raw material.

The plurality of modular raw materials may be disposed on the micro flow path P of the microfluidic chip 400.

In the present embodiment, the first modular raw material D1 dissolved in the first fluid, and the second modular raw material D2 dissolved in the second fluid will be disposed in the microfluidic chip 400, will be described by way of example.

Here, the first modular raw material D1 is a substance soluble in water and may be formed into a solid (e.g., powder block) or gel phase.

Further, the second modular raw material D2 is a substance soluble in oil and may be formed into a solid (e.g., powder block) or gel phase.

However, the first modular raw material D1 and the second modular raw material D2 are not limited to this and may comprise fluid surrounded in a solid or gel phase. In this case, each of the solid or gel phase surrounding the fluid is dissolved in the first fluid and the second fluid, and the first fluid may be mixed with the first modular raw material D1, or the second fluid may be mixed with the second modular raw material D2.

The micro flow path P of the microfluidic chip 400 may be formed to have different structures depending on the first modular raw material D1 and the second modular raw material D2 included in the microfluidic chip 400.

For example, the first fluid and the first modular raw material D1 dissolved in the first fluid may comprise water, a polyol, a thickener, a surfactant, an auxiliary emulsifier, a moisturizing agent, a salt, a preservative, an aqueous phase (e.g., dissolved in water) functional ingredient, and the like.

Here, the polyol may be understood as a multifunctional alcohol having two or more hydroxyl groups (-OH) in a molecule. The polyol may include glycol having two hydroxyl groups, glycerol having three hydroxyl groups, and pentaerythritol having four hydroxyl groups.

For example, the polyol may include at least one selected from the group consisting of ethylene glycol, propylene glycol, dipropylene glycol, 1,3-butylene glycol, diglycerin, 1,3-propanediol, glycerin, methylpropanediol, ethylhexanediol, 1,2-hexanediol, isoprene glycol, ethylalcohol, pentylene glycol, and isopentyldiol.

The thickener may include at least one selected from the group consisting of Gelatin, Gellan Gum, Guar Gum, Xanthan Gum, Locust Bean Gum, Alginic acid, Arabic Gum, Carrageenan, Pectin, Cellulose, Mannan, Carbomer, Sodium Polyacrylate, Polyacrylic Acid, Polyacrylate crosspolymer, Hydroxyethyl acrylate/Sodium acryloyldimethyl taurate Copolymer, Ammonium acryloyldimethyltaurate/vp copolymer, Sodium polyacryloyldimethyl taurate, Acrylate/C10-30 Alkyl acrylate crosspolymer, Ammonium acryloyldimethyltaurate/beheneth-25 methacrylate crosspolymer, Poly vinyl alcohol, and glyceryl acrylate/acrylic acid copolymer.

Also, for example, the second fluid and the second modular raw material D2 dissolved in the second fluid may include oil, a surfactant, a co-emulsifier, a functional raw material dissolved in the oil, and the like.

Additionally, at least one of the first fluid, the first modular raw material D1 dissolved in the first fluid, the second fluid, and the second modular raw material D2 dissolved in the second fluid may include at least one of moisturizing agent, surfactant, thickener, and auxiliary emulsifier.

The moisturizing agent may include at least one selected from a group consisting of Trehalose, Fructose, Sucrose, Sorbitol, Maltitol, Panthenol, Raffinose, Urea, Betaine, Glycereth-26, Methyl Gluceth-20, PEG-8, PEG-32, PEG-75, PEG/PPG/POLYBUTYLENE GLYCOL-8/5/3 GLYCERIN, ethylene glycol, propylene glycol, dipropylene glycol, 1,3-butylene glycol, diglycerin, 1,3-propanediol, glycerin, methylpropanediol, ethylhexanediol, 1,2-hexanediol, isoprene glycol, ethylalcohol, pentylene glycol, and isopentyldiol. The surfactant may include at least one selected from the group consisting of Sorbitan Monostearate, Sorbitan Sesquioleate, Polysorbate 20, Polysorbate 60, Polysorbate 80, PEG-40 Stearate, PEG-100 Stearate, PEG-60 Glyceryl Isostearate, PEG 75- Stearate, Ceteth-20, Steareth-20, Cetearyl Glucoside, Arachidyl Glucoside, Lauryl Glucoside, Sucrose Polystearate, Sucrose Laurate, Polyglyceryl-3 Methylglucose Distearate, Polyglyceryl-10 Stearate, Lecithin, Sodium Stearoyl Glutamate, Glyceryl Stearate Citrate, Potassium Cetyl Phosphate, Sodium Methyl Stearoyl Taurate, Inulin Lauryl Carbamate, Ceteareth-12, PEG-5 Rapeseed Sterol, Methoxy PEG-114/Polyepsion Caprolactone, Cetyl PEG/PPG 10/1 Dimethicone Triglyceride, PEG-10 Dimethicone, Lauryl PEG-9 Polydimethylsiloxyethyl Dimethicone, Polyglyceryl-2 Dipolyhydroxystearate, PEG-60 Hydrogenated Castor Oil, PEG-30 Hydrogenated Castor Oil, Octyldodeceth-16, Octyldodeceth-25, Bis-PEG-18 Methyl Ether Dimethyl Silane, Polyglyceryl-6 Caprylate, Polyglyceryl-4 Caprate.

The auxiliary emulsifier included in the first content may include at least one selected from a group consisting of Cetearyl Alcohol, Cetyl Alcohol, Stearyl Alcohol, Behenyl Alcohol, Glyceryl Stearate, Stearic Acid.

However, these are merely exemplary components and the types of components included in the first fluid, the first modular raw material D1 dissolved in the first fluid, the second fluid, and the second modular raw material D2 dissolved in the second fluid are not limited to them.

The first fluid, the first modular raw material D1 dissolved in the first fluid, the second fluid, and the second modular raw material D2 dissolved in the second fluid may pass through the micro flow path P formed in the inlet portion 410, the dissolution portion 420, the confluence 430, the stir portion 440, the vortexing portion 450, and a discharge portion 460 of the microfluidic chip 400, and may be mixed and emulsified to be produced as an O/W emulsion or a W/O emulsion.

The microfluidic chip 400 may be understood as a composition in which the micro plow path P is formed inside a plate T provided in a flat plate shape. As such, by placing the micro flow path P on the singular flat plate T, the cosmetic manufacturing apparatus 10 may be miniaturized.

A cross section of the micro flow path P formed inside the microfluidic chip 400 may be rectangular, and in this case average length of each side may be from 0.5mm to 1mm. In a case of the micro flow path P having a circular cross section, average length of diameter may be from 0.5mm to 1mm.

In a case of the microfluidic chip 400 forming the micro flow path P inside, the speed of the fluid may be accelerated and increases efficiency of mixing and emulsification.

The micro flow path may comprise the first micro flow path P1 and the second micro flow path P2 formed on the dissolution portion 420, the third micro flow path P3 formed on the confluence 430, the fourth micro flow path P4 formed on the stir portion 440, the vortexing portion 450 formed on the vortexing portion 450, and a discharge flow path 442 formed on the discharge portion 460.

Meanwhile, in the present embodiment, the micro flow path P formed in the microfluidic chip 400 may constitute a single layer path. The single layer path may be understood as a path in which height difference of flow paths is not involved in mixing and emulsification of each fluid or emulsification of the mixed fluid during the mixing and emulsification of fluid.

The single layer path in the present embodiment may correspond to the first micro flow path P1, the second micro flow path P2, the third micro flow path P3, the fourth micro flow path P4, the fifth micro flow path P5, and the discharge flow path 442 implemented in the flat plate T.

However, the present embodiment is not limited to such, and the second micro flow path P2, the third micro flow path P3, the fourth micro flow path P4, the fifth micro flow path P5, and the discharge flow path 442 are disposed on a same plane, but the middle portion of the first micro flow path P1b in which the first modular raw material D1 is disposed, and the middle portion of the second micro flow path P2b in which the second modular raw material D2 is disposed in the first micro flow path P1 may be disposed in a different micro flow path and a different plane (refer to FIG. 4).

The microfluidic chip 400 may comprise the inlet portion 410, the dissolution portion 420, the confluence 430, the stir portion 440, the vortexing portion 450, and the discharge portion 460.

In the present embodiment, the first fluid flows along the first micro flow path P1, the third micro flow path P3, the fourth micro flow path P4, and the fifth micro flow path P5, and the second fluid flows along the second micro flow path P2, the third micro flow path P3, the fourth micro flow path P4, and the fifth micro flow path P5. At this time, the first fluid dissolves the first modular raw material D1 in the first micro flow path P1, and the second fluid dissolves the second modular raw material D2 in the second micro flow path P2.

The inlet portion 410 may comprise a first fluid inlet 411 through which the first fluid is introduced; and a second fluid inlet 412 through which the second fluid is introduced.

At least one of the first fluid inlet 411 and the second fluid inlet 412 may be provided in a plurality.

In the present embodiment, the first fluid inlet 411 will be provided in a plurality, and the second fluid inlet 412 will be provided in a singularity, will be described by way of example.

The first fluid inlet 411 may comprise a first upper inlet 411a, a second upper inlet 411b, a first lower inlet 411c, and a second lower inlet 411d.

The first fluid introduced through each of the first upper inlet 411a, the second upper inlet 411b, the first lower inlet 411c, and the second lower inlet 411d may meet in the upstream of the first micro flow path P1 and move to the downstream.

In the present embodiment, a point at which the flow of the fluid started is the upstream, and a point at which the flow of the fluid ended is the downstream based on the direction of the flow of the fluid. For example, the first micro flow path P1 may be disposed on the upper part of the microfluidic chip 400 above the second micro flow path P2 to the fifth micro flow path P5.

The dissolution portion 420 may comprise the first micro flow path P1 through which the first fluid moves, and the second micro flow path P2 through which the second fluid moves.

The first modular raw material D1 is disposed in the first micro flow path P1, and the second modular raw material D2 is disposed in the second micro flow path P2. In other words, the first modular raw material D1 is pre-loaded in the first micro flow path P1, and the second modular raw material D2 is pre-loaded in the second micro flow path P2.

In the first micro flow path P1, the first fluid converges with the first modular raw material D1, and the first modular raw material D1 is dissolved by the first fluid.

In the second micro flow path P2, the second fluid converges with the second modular raw material D2, and the second modular raw material D2 is dissolved by the second fluid.

The first micro flow path P1 may comprise an upstream of the first micro flow path P1a through which the first fluid is introduced; the middle portion of the first micro flow path P1b in which the first modular raw material D1 is disposed; and a downstream of the first micro flow path P1c through which the first fluid and the first modular raw material D1 dissolved by the first fluid flows.

Here, the middle portion of the first micro flow path P1b in which the first modular raw material D1 is disposed may be disposed on a different plane from the first upstream of the first micro flow path P1a and the first downstream of the first micro flow path P1c. That is, the first middle portion of the first micro flow path P1b may be disposed below or above the first upstream of the first micro flow path P1a and the first downstream of the first micro flow path P1c (refer to FIG. 4).

The second micro flow path P2 may comprise an upstream of the second micro flow path P2a through which the second fluid is introduced; the second middle portion of the second micro flow path P2b in which the second modular raw material D2 is disposed; and a second downstream of the first micro flow path P2c through which the second fluid and the second modular raw material D2 dissolved by the second fluid flows.

Likewise, the middle portion of the second micro flow path P2b in which the second modular raw material D2 is disposed may be disposed on a different plane from the second upstream of the second micro flow path P2a and the second downstream of the first micro flow path P2c. That is, the second middle portion of the second micro flow path P2b may be disposed below or above the second upstream of the second micro flow path P2a and the second downstream of the first micro flow path P2c.

The first micro flow path P1 may comprise the first middle portion of the first micro flow path P1b in which the first modular raw material D1 is disposed, and the cross-sectional area of the middle portion of the first micro flow path P1b through which the first fluid flows may be altered as the first modular raw material D1 is dissolved by the first fluid (refer to FIG. 4).

(a) of FIG. 4 conceptually shows that the cross-sectional area of the middle portion of the first micro flow path P1b is relatively small at a point where the first fluid had started flowing, and (b) of FIG. 4 is a point at which the cross-sectional area of the middle portion of the first micro flow path P1b is relatively bigger a certain period of time had passed after the first fluid had flown.

Further, the second micro flow path P2 may comprise the second middle portion of the second micro flow path P2b in which the second modular raw material D2 is disposed, and the cross-sectional area of the middle portion of the second micro flow path P2b through which the second fluid flows may be altered as the second modular raw material D2 is dissolved by the second fluid.

That is, the first micro flow path P1, the second micro flow path P2, the third micro flow path P3, the fourth micro flow path P4, and the fifth micro flow path P5 may be formed on an identical plane, but the middle portion of the first micro flow path P1b and the middle portion of the second micro flow path P2b may be formed on a different plane from the first micro flow path P1, the second micro flow path P2, the third micro flow path P3, the fourth micro flow path P4, and the fifth micro flow path P5.

The second micro flow path P2, the third micro flow path P3, the fourth micro flow path P4, and the fifth micro flow path P5 are formed on an identical plane, but the middle portion of the first micro flow path P1b of the first micro flow path P1 in which the first modular raw material D1 is disposed, and the middle portion of the second micro flow path P2b of the second micro flow path P2 in which the second modular raw material D2 is disposed, may be formed on a different plane from the second micro flow path P2 to the fifth micro flow path P5.

Further, the first micro flow path P1 to the fifth micro flow path P5 are formed inside the flat shaped plates T, and a protrusion portion T1 protruding toward the first micro flow path P1 and the second micro flow path P2 may be provided on the plate T (refer to FIG. 4).

In specific, the protrusion portion T1 is disposed protruding toward the middle portion of the first micro flow path P1b in which the first modular raw material D1 is disposed and toward the middle portion of the second micro flow path P2b in which the second modular raw material D2 is disposed.

By such, as the first fluid is collided with the protrusion portion T1, and as the direction of the flux of the first fluid is converted to face the first modular raw material D1, the first modular raw material D1 can be readily dissolved. Likewise, as the second fluid is collided with the protrusion portion T1, and as the direction of the flux of the second fluid is converted to face the second modular raw material D2, the second modular raw material D2 can be readily dissolved.

Further, a concave portion T2 providing space for the first modular raw material D1 and the second modular raw material D2 to settle may be provided in the plate T.

In specific, the concave portion T2 in which the first modular raw material D1 can be settled, may be provided on one side of the first micro flow path P1, and the concave portion T2 in which the second modular raw material D2 can be settled, may be provided on one side of the second micro flow path P2 (refer to FIG. 4).

The concave portion T2 may be disposed below the first micro flow path P1 and the second micro flow path P2.

Here, a lower portion may be understood as a direction described based on the vertical cross section of the microfluidic chip 400 regarding the direction extending from the inlet portion 410 to the discharge portion 460.

The concave portion T2 and the protrusion portion T1 may be disposed facing toward each other. That is, the protrusion portion T1 may be formed protruding towards the concave portion T2.

The confluence 430 may comprise the third micro flow path P3 in which the first fluid, the first modular raw material D1 dissolved by the first fluid, the second fluid, and the second modular raw material D2 dissolved by the second fluid converge.

The stir portion 440 may comprise the fourth micro flow path P4 provided in a zigzag shape.

The fourth micro flow path P4 extends from the third micro flow path P3 formed in the confluence 430 and may comprise a first mixing flow path 4411 formed to be extended in one direction; a second mixing flow path 4412 bent at an angle (e.g., 60 degrees to 120 degrees) preset by the first mixing flow path 4411; and a third mixing flow path 4413 bent at an angle (e.g., 60 degrees to 120 degrees) preset by the second mixing flow path 4412.

In the fourth micro flow path P4 of the stir portion 440, the first fluid, the first modular raw material D1 dissolved in the first fluid, the second fluid, and the second modular raw material D2 dissolved in the second fluid may be mixed to form a mixed fluid.

Here, the first mixing flow path 4411 and the third mixing flow path 4413 may be formed parallel to each other.

The width W3 of the first mixing flow path 4411 and the width W5 of the third mixing flow path 4413 may increase and then decrease along the flow direction of the fluid, and the width W4 of the second mixing flow path 4412 may be formed to be constant.

In the present embodiment, the fourth micro flow path P4 may comprise a plurality of first mixing flow path 4411, a plurality of second mixing flow path 4412, and a plurality of third mixing flow path 4413. For example, the fourth micro flow path P4 may comprise three first mixing flow paths 4411, two third mixing flow paths 4413, and four second mixing flow paths 4412.

FIG. 6 shows a schematic view of flux of the fluid (mixed fluid) occurring in the stir portion 440.

The stir portion 440 comprises the fourth micro flow path P4 which repeats increasing and decreasing in its width, and while the highly concentrated mixed fluid located at B introduced in advance is rotating back and forth in the fourth micro flow path P4, the mixed fluid located in B and the mixed fluid located in A are mixed, becomes the average concentration level of the mixed fluid in B and the mixed fluid in C and moves to location C. By this, the first fluid, the dissolved modular raw material D1, the second fluid, and the dissolved second modular raw material D2 can be thoroughly mixed as a whole.

The vortexing portion 450 may comprise the fifth micro flow path P5 in which a plurality of vortex forming flow paths 451 causing vortices by altering the direction of the flow of the fluid (or the mixed fluid) is provided.

The mixed fluid is broken into particles by vortex formed by the fifth micro flow path P5, and an emulsion comprising emulsion particles which decrease in size as they proceed to downstream may be formed.

For example, an emulsion particle flowing in a second area A2 of the vortexing portion 450 is provided in a smaller size than an emulsion particle flowing in a first area A1 of the vortexing portion 450.

The vortexing portion 450, in order for the first fluid, the dissolved first modular raw material D1, the second fluid, and the second modular raw material D2 to be emulsified to form an emulsion, may form the plurality of vortex forming flow paths 451 forming a vortex in a flux by altering the flow direction of the fluid.

Here, emulsification may be understood as a technique for dispersing one of two immiscible fluids such as oil and water into small particles and placing them into the other fluid in a stable state.

The vortex forming flow path 451 is sequentially formed in a plurality in the first area A1 of the vortexing portion 450.

Further, the vortex forming flow path 451 is sequentially formed in a plurality in the second area A2 below the first area A1.

The vortex forming flow path 451 formed in the first area A1 and the vortex forming flow path 451 formed in the second area A2 may be connected through a connection flow path 452, and the connection flow path 452 may be formed in a shape of a straight flow path.

In the present embodiment, the vortex forming flow path 451 formed to rotate the entering fluid into one direction (anti-clockwise to the drawing in the present embodiment) and rotates again in the other direction (clockwise to the drawing in the present embodiment), will be described by way of example.

In specific, each of the vortex forming flow path 451 may comprise a first turning flow path 4511 which guides the entering fluid to rotate in one direction; a second turning flow path 4512 which guides the fluid rotating in one direction to rotate in the other direction; and a direction conversion flow path 4513 which converts the direction of the fluid's rotation between the first turning flow path 4511 and the second turning flow path 4512.

Here, the first fluid, the dissolved first modular raw material D1, the second fluid, and the dissolved second modular raw material D2 may pass through the plurality of vortex forming flow paths 451 and the emulsification may proceed.

The external phase fluid (first fluid and dissolved first modular raw material D1, or second fluid and dissolved second modular raw material D2) and the internal phase fluid (second fluid and dissolved second modular raw material D2, or first fluid and dissolved first modular raw material D1) may become thinned or be broken due to the formation of a vortex while passing through the vortex forming flow path 451.

By repeating this process, finally, an emulsion in which the finely broken internal phase fluid is stably present inside the external fluid can be formed.

The discharge portion 460 may extend from the vortexing portion 450.

The discharge portion 460 may comprise a discharge flow path 462 extending from the vortex forming flow path 451 of the vortexing portion 450; and a discharge hole 464 through which the produced emulsion is discharged.

The discharge flow path 462 may be formed to extend in one direction after being bent from the vortex forming flow path 451 towards one side at a preset angle (e.g., 90 degrees).

The discharge flow path 462 may comprise a portion with width that increases as it proceeds from the vortex forming flow path 451 to the discharge hole 464.

The discharge portion 460 may be formed on one end of the discharge flow path 462.

Hereinafter, the cosmetic manufacturing apparatus 10 configured to operate the microfluidic chip 400 will be described in detail.

The cosmetic manufacturing apparatus 10 may comprise the storage portion 100 configured to store the first fluid and the second fluid; the above-mentioned microfluidic chip 400 configured to receive the first fluid and the second fluid from the storage portion; and the main body 300 accommodating the storage portion 100 and the microfluidic chip 400.

Here, the microfluidic chip 400 may be replaceably provided regarding the main body 300.

The storage portion 100 may comprise a first storage portion 110 configured to store the first fluid; and a second storage portion 120 configured to store thee second fluid which is different from the first fluid. Here, in this embodiment, water is provided as a hydrophilic fluid, and oil is provided as a hydrophobic fluid, will be described by way of example.

The first storage portion 110 and the second storage portion 120 may have their capacities corresponding to the amounts of the first fluid and the second fluid, respectively, which are completely discharged by one time operation. However, the technical idea of the present disclosure is not limited to this, but it may also be contemplated that the first storage portion 110 and the second storage portion 120 have capacities corresponding to the amounts of the first fluid and the second fluid, respectively, which are completely discharged by multi-time operations.

Further, the first storage portion 110 and the second storage portion 120 may also be provided replaceably.

Below the storage portion 100, a coupling portion 200 may be provided, which connects the microfluidic chip 400 and the storage portion 100.

In specific, below the first storage portion 110, a first coupling portion 210 may be provided, which connects the first fluid inlet 411 of the microfluidic chip 400 with the first storage portion 110 to deliver the first fluid stored in the first storage portion 110 to the micro flow path P of the microfluidic chip 400.

Further, below the second storage portion 120, a second coupling portion 220 may be provided, which connects the second fluid inlet 412 of the microfluidic chip 400 with the second storage portion 120 to deliver the second fluid stored in the second storage portion 120 to the micro flow path P of the microfluidic chip 400.

The microfluidic chip 400 may be disposed below the coupling portion 200.

The first coupling portion 210 may comprise a first coupling flow path 212, a second coupling flow path 214, a third coupling flow path 216, and a fourth coupling flow path 218, which are connected to the first storage portion 110.

In specific, the first coupling flow path 212 may be coupled to the first upper inlet 411a, the second coupling flow path 214 may be coupled to the second upper inlet 411b of the microfluidic chip 400, the third coupling flow path 216 may be coupled to the first lower inlet 411c of the microfluidic chip 400, and the fourth coupling flow path 218 may be coupled to the second lower inlet 411d of the microfluidic chip 400.

Further, the second coupling portion 220 may be coupled to the second fluid inlet 412 of the microfluidic chip 400.

This embodiment will be described by way of example under the condition that the first fluid stored in the first storage portion 110 is discharged to the first fluid inlet 411 of the inlet portion 410 through the four coupling flow paths 212, 214, 216, 218, and that the second fluid stored in the second storage portion 120 is discharged to the second fluid inlet 412 of the inlet portion 410 through one coupling flow path. However, the number of coupling flow paths of the present disclosure is not limited to this.

In the present embodiment, the microfluidic chip 400 and the coupling portion 200 coupled thereto may be disposed perpendicular to each other.

That is, the micro flow path P of the microfluidic chip 400 and the coupling portion 200 may form an angle of 90 degrees.

For example, the micro flow path P of the microfluidic chip 400 may be disposed parallel to the bottom surface (XY plane) of the main body 300when inserted into the main body 300, and the coupling portion 200 may be disposed perpendicular to the bottom surface (in the Z-axis direction) of the main body 300.

However, the technical idea of the present invention is not limited to this, and the microfluidic chip 400 and the coupling portion 200 may be disposed parallel to each other.

In specific, the micro flow path P of the microfluidic chip 400 may be disposed in the direction of the gravity (in the Z-axis direction), and the fluid introduced through the inlet portion 410 of the microfluidic chip 400 may be mixed and emulsified by the gravity and then discharged to the outside through the discharge hole 464 of the discharge portion 460.

The main body 300 may comprise a first main body 310 in which a first insertion hole (not illustrated in drawings) is formed to provide a space for the microfluidic chip 400 to be inserted; and a second main body 320 in which a second insertion hole 130 is formed to provide a space for the storage portion 100 to be inserted.

Although the present embodiment is described under the condition that the first main body 310 and the second main body 320 are separately installed, the first main body 310 and the second main body 320 may be integrally formed as one member.

The first insertion hole (not illustrated in drawings) into which the microfluidic chip 400 may be inserted can be formed in the first main body 310. Here, the first insertion hole (not illustrated in drawings) may be a space into which the microfluidic chip 400 may be inserted.

Further, an operation button 330 may be disposed on one side of the main body 300, which initiates an operation by which the fluid stored in the storage portion 100 is moved to the microfluidic chip 400, and then mixed and emulsified with the modular raw material disposed on the microfluidic chip 400 to form an emulsion.

Further, a recognition portion (not illustrated in drawings) configured to recognize the types of the microfluidic chip 400 may be provided in the first insertion hole (not illustrated in drawings) of the main body 300.

When the kind of microfluidic chip 400 is recognized by the recognition portion (not illustrated in drawings), a driving portion (not illustrated in drawings) controlled by a control portion (not illustrated in drawings) may apply pressure corresponding to the kind of the microfluidic chip 400 onto the micro flow path P.

The second insertion hole 130 into which the first storage portion 110 and the second storage portion 120 are inserted may be formed in the second main body 320.

In the present embodiment, it has been illustrated that the second insertion hole 130 is formed in one space. However, the technical idea of the present disclosure is not limited to this, and the second insertion hole 130 may be formed in a plurality of spaces so that each can accommodate the first storage portion 110 and the second storage portion 120.

The second main body 320 may be disposed above the first main body 310.

The microfluidic chip 400 may be provided to be separable from the main body 300.

The microfluidic chip 400 may receive the first fluid in the first storage portion 110 and the second fluid in the second storage portion 120.

After the first fluid dissolves the first modular raw material D1 and the second fluid dissolves the second modular raw material D2 on the micro flow path P of the microfluidic chip 400, the first fluid, the dissolved first modular raw material D1, the second fluid, and the dissolved second modular raw material D2 may be mixed and emulsified to form an emulsion, and then may be discharged from the microfluidic chip 400 to the outside.

In specific, the type of the emulsion (e.g., W/O emulsion or O/W emulsion) may be decided depending on the ratio of the first fluid and the second fluid supplied from the storage portion 100 to the microfluidic chip 400.

The ratio of the first fluid and the second fluid supplied to the microfluidic chip 400 may be adjusted by the discharge amounts of the first fluid and the second fluid discharged from the first storage portion 110 and the second storage portion 120, and the discharge amounts of the first fluid and the second fluid may be controlled by the control portion (not illustrated in drawings).

For example, when the supply amount of water which is the first fluid is greater than the supply amount of oil which is the second fluid, an O/W emulsion is produced, and when the supply amount of oil which is the second fluid is greater than the supply amount of water which is the first fluid, a W/O emulsion is produced.

Further, the cosmetic manufacturing apparatus 10 may further comprise a cosmetic container 500 disposed below the discharge hole 464 of the microfluidic chip 400.

The cosmetic container 500 may store the emulsion discharged from the discharge hole 464.

Further, the cosmetic container 500 may be optionally provided to be fixed to the microfluidic chip 400.

In specific, the inlet of the cosmetic container 500 and the discharge hole 464 of the microfluidic chip 400 may be disposed in alignment, and the inlet of the cosmetic container 500 and the discharge hole 464 may be fixed to each other.

In this case, the cosmetic container 500 and the microfluidic chip 400 remain fixed when the emulsion is being discharged from the microfluidic chip 400, and when the emulsion is completely discharged from the microfluidic chip 400, the cosmetic container 500 may be removed from the microfluidic chip 400.

Further, the cosmetic manufacturing apparatus 10 may further comprise the driving portion (not illustrated in drawings) configured to provide pressure causing the fluid and the modular raw material dissolved in the fluid to flow along the micro flow path P formed in the microfluidic chip.

In specific, the driving portion (not illustrated in drawings) may provide pressure by which the first fluid, the dissolved first modular raw material D1, the second fluid, and the dissolved second modular raw material D2 flow along the micro flow path P.

The driving portion (not illustrated in drawings) may apply pressure toward the micro flow path P formed from the first fluid inlet 411 and the second fluid inlet 412 to the discharge hole 464.

The driving portion (not illustrated in drawings) may apply pressure from the first fluid inlet 411 and the second fluid inlet 412, along the micro flow path P and to the discharge hole 464 by inserting air into the first fluid inlet 411 and the second fluid inlet 412.

Further, the cosmetic manufacturing apparatus 10 may comprise the control portion (not illustrated in drawings) configured to control the pressure applied from the driving portion (not illustrated in drawings) on the micro flow path P of the microfluidic chip 400.

The control portion (not illustrated in drawings) may control the flow rate of the fluid flowing along the micro flow path P.

In specific, the control portion (not illustrated in drawings) may control the flow rate of the first fluid flowing along the first micro flow path P1, the flow rate of the second fluid flowing along the second micro flow path P2, the flow rate of the mixed fluid flowing along the fourth micro flow path P4, and the flow rate of the mixed fluid flowing along the fifth micro flow path P5.

The control portion (not illustrated in drawings) may control the pressure applied to the micro flow path P differently depending on the type of the microfluidic chip 400.

For example, the control portion (not illustrated in drawings) can apply a first pressure on the micro flow path P when a first type of microfluidic chip 400 is provided, and the control portion (not illustrated in drawings) can apply a second pressure on the micro flow path P when a second type of microfluidic chip 400 is provided.

Here, the first type of microfluidic chip 400 may comprise the first modular raw material D1 comprising a first ingredient and the second modular raw material D2 comprising a second ingredient, and the second type of microfluidic chip 400 may comprise the first modular raw material D1 comprising a third ingredient and the second modular raw material D2 comprising a fourth ingredient. Here, the first ingredient, the second ingredient, the third ingredient, and the fourth ingredient may be different from each other.

Further, the control portion (not illustrated in drawings) may control the discharge amount of the first fluid stored in the first storage portion 110, which is discharged to the microfluidic chip 400, and the discharge amount of the second fluid stored in the second storage portion 120, which is discharged to the microfluidic chip 400.

Hereinafter, the operation process of the cosmetic manufacturing apparatus 10 will be described.

The control method of the cosmetic manufacturing apparatus according to the present embodiment may comprise: a step S1 in which one type of microfluidic chip 400 is inserted into the main body 300; a step S2 in which the type of the microfluidic chip 400 is recognized by the recognition portion (not illustrated in drawings), and a preset pressure corresponding to the type of the microfluidic chip is applied to the micro flow path P; a step S3 in which the first fluid dissolves the first modular raw material D1 disposed in the dissolution portion 420, the second fluid dissolves the second modular raw material D2 disposed in the dissolution portion 420, and converges in the third micro flow path P3; a step S4 in which the first fluid, the dissolved modular raw material D1, the second fluid, and the dissolved second modular raw material D2 are mixed while proceeding along the fourth micro flow path P4 formed on the stir portion 440; a step S5 in which first fluid, the dissolved first modular raw material D1, the second fluid, and the dissolved second modular raw material D2 are mixed to form a cosmetic while proceeding along the vortex forming flow path 451 formed on the vortexing portion 450; and a step S6 in which the cosmetic is discharged to outside through the discharge hole 464 of the discharge portion 460.

## Claims

1. A microfluidic chip comprising:
an inlet portion (410) comprising a first fluid inlet (411) through which a first fluid is introducible and a second fluid inlet (412) through which a second fluid is introducible;
a dissolution portion (420) comprising a first micro flow path (P1) in which a first modular raw material (D1) to be dissolved by the first fluid is pre-loaded and a second micro flow path (P2) in which a second modular raw material (D2) to be dissolved by the second fluid is pre-loaded;
a confluence (430) comprising a third micro flow path (P3) in which the first fluid, the first modular raw material (D1) dissolved by the first fluid, the second fluid, and the second modular raw material(D2) dissolved by the second fluid can converge;
a stir portion (440) comprising a fourth micro flow path (P4) which is configured to form a mixed fluid by mixing the first fluid, the first modular raw material (D1) dissolved by the first fluid, the second fluid, and the second modular raw material (D2) dissolved by the second fluid; and
a vortexing portion (450) configured to form a cosmetic content comprising particles by creating a vortex and cutting off the mixed fluid,
wherein the fourth micro flow path (P4) provided on the stir portion (440) comprises:
a first mixing flow path (4411) which extends from the third micro flow path(P3) formed on the confluence (430) and extends in one direction;
a second mixing flow path (4412) ); and
a third mixing flow path (4413), and
wherein at least one of the first mixing flow path (4411) and the third mixing flow path (4413) is formed in a figure which increases and then decreases along the direction of a fluid flow,
**characterized in that**
the second mixing flow path (4412) is bent at a preset angle from the first mixing flow path (4411) and the third mixing flow path (4413) is bent at a preset angle from the second flow path (4412).

2. The microfluidic chip of claim 1, wherein the first fluid introduced in the first fluid inlet (411) passes through the first micro flow path (P1) and dissolves the first modular raw material (D1),
wherein the second fluid introduced in the second fluid inlet (412) passes through the second micro flow path(P2) and dissolves the second modular raw material (D2).

3. The microfluidic chip of claim 1, wherein the first fluid is provided as a hydrophilic fluid,
wherein the second fluid is provided as a hydrophobic fluid,
wherein the first modular raw material (D1) is formed in a solid phase or gel phase which can be dissolved in the hydrophilic fluid, and
wherein the second modular raw material (D2) is formed in a solid phase or gel phase which can be dissolved in the hydrophobic fluid.

4. The microfluidic chip of claim 1, wherein a fifth micro flow path (P5) provided on the vortexing portion (450) includes a plurality of vortex forming flow paths (451) configured to create a vortex by converting the traveling direction of the fluid.

5. The microfluidic chip of claim 4, wherein each of the vortex forming flow path (451) comprises:
a first turning flow path (4511) which directs the entering mixed fluid to rotate in one direction;
a second turning flow path (4512) which directs the mixed fluid rotating in one direction to rotate in the other direction; and
a direction conversion flow path (4513) which converts the rotation direction of the mixed fluid between the first turning flow path (4511) and the second turning flow path (4512).

6. The microfluidic chip of claim 4, wherein the width of the first micro flow path (P1) in which the first modular raw material is disposed, or the width of the second micro flow path (P2) where the second modular raw material is disposed are to be formed bigger than the width of the fifth micro flow path (P2).

7. The microfluidic chip of claim 1, wherein the particles formed at the vortexing portion (450) are emulsion particles, and the cosmetic content comprising the emulsion particles is provided as an emulsion.

8. The microfluidic chip of claim 1, wherein the first micro flow path (P1) comprises a middle portion of the first micro flow path (P1b) in which the first modular raw material (D1) is disposed,
the cross-sectional area of the middle portion of the first micro flow path (P1b) in which the first fluid flows changes as the first modular raw material (D1) is dissolved by the first fluid,
the second micro flow path (P2) comprises a middle portion of the second micro flow path (P2b) in which the second modular raw material (D2) is disposed, and
the cross-sectional area of the middle portion of the second micro flow path (P2b) in which the second fluid flows changes as the second modular raw material (D2) is dissolved by the second fluid.

9. The microfluidic chip of claim 1, wherein the second micro flow path (P2), the third micro flow path (P3), the fourth micro flow path(P4), and a fifth micro flow path (P5) are formed on an identical plane, but a middle portion of the first micro flow path (P1b) of the first micro flow path (P1) in which the first modular raw material (D1) is disposed and a middle portion of the second micro flow path (P2b) of the second micro flow path (P2) in which the second modular raw material (D2) is disposed are formed on a different plane from the second micro flow path (P2) to the fifth micro flow path (P5).

10. The microfluidic chip of claim 1, wherein the first micro flow path (P1) to a fifth micro flow path (P5) are formed inside a plane shape plate (T), and
the plate (T) is provided with a protrusion portion (T1) which is protruded toward the first micro flow path (P1) and the second micro flow path (P2).

11. A cosmetic manufacturing apparatus comprising a storage portion (100) configured to store a first fluid and a second fluid;
a microfluidic chip (400) according to claim 1 which receives the first fluid and the second fluid from the storage portion (100);
a main body (300) configured to accommodate the storage portion (100) and the microfluidic chip (400); and
the microfluidic chip (400) is replaceably provided regarding the main body (300).

## Patentansprüche

1. Ein mikrofluidischer Chip, der aufweist:
einen Einlassabschnitt (410), der einen ersten Fluideinlass (411), durch den ein erstes Fluid einleitbar ist, und einen zweiten Fluideinlass (412), durch den ein zweites Fluid einleitbar ist, aufweist,
einen Auflösungsabschnitt (420), der einen ersten Mikro-Strömungspfad (P1), in dem ein erstes modulares Rohmaterial (D1), das mittels des ersten Fluids gelöst werden soll, vorab geladen ist, und einen zweiten Mikro-Strömungspfad (P2), in dem ein zweites modulares Rohmaterial (D2), das mittels des zweiten Fluids gelöst werden soll, vorab geladen ist, aufweist,
eine Zusammenströmungsstelle (430), die einen dritten Mikro-Strömungspfad (P3) aufweist, in dem das erste Fluid, das mittels des ersten Fluids gelöste erste modulare Rohmaterial (D1), das zweite Fluid und das mittels des zweiten Fluids gelöste zweite modulare Rohmaterial (D2) zusammenströmen können,
einen Bewegungsabschnitt (440), der einen vierten Mikro-Strömungspfad (P4) aufweist, der konfigurierst ist, um mittels Mischens des ersten Fluids, des mittels des ersten Fluids gelösten ersten modularen Rohmaterials (D1), des zweiten Fluids und des mittels des zweiten Fluids gelösten zweiten modularen Rohmaterials (D2) ein gemischtes Fluid zu bilden, und
einen Wirbelabschnitt (450), der konfiguriert ist, um einen kosmetischen Inhalt, der Partikeln aufweist, zu bilden mittels Erzeugens eines Wirbels und Abtrennens des gemischten Fluids,
wobei der vierte Mikro-Strömungspfad (P4), der an dem Bewegungsabschnitt (440) bereitgestellt ist, aufweist:
einen ersten Misch-Strömungspfad (4411), der sich von dem an der Zusammenströmungsstelle (430) gebildeten dritten Mikroströmungspfad (P3) erstreckt und sich in eine Richtung erstreckt,
einen zweiten Misch-Strömungspfad (4412) und
einen dritten Misch-Strömungspfad (4413) und
wobei mindestens einer des ersten Misch-Strömungspfads (4411) und des dritten Misch-Strömungspfads (4413) in einer Form ausgebildet sind, die entlang der Richtung einer Fluidströmung zunimmt und dann abnimmt,
**dadurch gekennzeichnet, dass**
der zweite Misch-Strömungspfad (4412) in einem vorbestimmten Winkel vom ersten Misch-Strömungspfad (4411) gebogen ist und der dritte Misch-Strömungspfad (4413) in einem vorbestimmten Winkel vom zweiten Strömungspfad (4412) gebogen ist.

2. Der mikrofluidische Chip gemäß Anspruch 1, wobei das in den ersten Fluideinlass (411) eingeleitete erste Fluid durch den ersten Mikro-Strömungspfad (P1) durchströmt und das erste modulare Rohmaterial (D1) auflöst,
wobei das in den zweiten Fluideinlass (412) einleitete zweite Fluid durch den zweiten Mikro-Strömungspfad (P2) durchströmt und das zweite modulare Rohmaterial (D2) löst.

3. Der mikrofluidische Chip gemäß Anspruch 1, wobei das erste Fluid als ein hydrophiles Fluid bereitgestellt ist,
wobei das zweite Fluid als ein hydrophobes Fluid bereitgestellt ist,
wobei das erste modulare Rohmaterial (D1) in einer festen Phase oder Gelphase gebildet ist, die in dem hydrophilen Fluid gelöst werden kann, und
wobei das zweite modulare Rohmaterial (D2) in einer festen Phase oder Gelphase gebildet ist, die in dem hydrophoben Fluid gelöst werden kann.

4. Der mikrofluidische Chip gemäß Anspruch 1, wobei ein fünfter Mikro-Strömungspfad (P5), der an dem Wirbelabschnitt (450) bereitgestellt ist, eine Mehrzahl von wirbelbildenden Strömungspfaden (451) aufweist, die konfiguriert sind, um mittels Umwandelns der Bewegungsrichtung des Fluids einen Wirbel zu erzeugen.

5. Der mikrofluidische Chip gemäß Anspruch 4, wobei jeder der wirbelbildenden Strömungspfade (451) aufweist:
einen ersten Dreh-Strömungspfad (4511), der das einströmende gemischte Fluid lenkt, um in eine Richtung zu rotieren,
einen zweiten Dreh-Strömungspfad (4512), der das in einer Richtung rotierende gemischte Fluid lenkt, um in die andere Richtung zu rotieren, und
einen Richtungsumwandlung-Strömungspfad (4513), der die Rotationsrichtung des gemischten Fluids zwischen dem ersten Dreh-Strömungspfad (4511) und dem zweiten Dreh-Strömungspfad (4512) umwandelt.

6. Der Mikrofluidik-Chip gemäß Anspruch 4, wobei die Breite des ersten Mikro-Strömungspfads (P1), in dem das erste modulare Rohmaterial angeordnet ist, oder die Breite des zweiten Mikro-Strömungspfads (P2), in dem das zweite modulare Rohmaterial angeordnet ist, größer gebildet ist als die Breite des fünften Mikro-Strömungspfads (P2).

7. Der mikrofluidische Chip gemäß Anspruch 1, wobei die am Wirbelabschnitt (450) gebildeten Partikel Emulsionspartikel sind und der die Emulsionspartikel aufweisende Kosmetikinhalt als eine Emulsion bereitgestellt ist.

8. Der mikrofluidische Chip gemäß Anspruch 1, wobei der erste Mikro-Strömungspfad (P1) einen mittleren Abschnitt des ersten Mikro-Strömungspfads (P1b) aufweist, in dem das erste modulare Rohmaterial (D1) angeordnet ist,
die Querschnittsfläche des mittleren Abschnitts des ersten Mikro-Strömungspfads (P1b), in dem das erste Fluid strömt, sich ändert, wenn das erste modulare Rohmaterial (D1) mittels des ersten Fluids aufgelöst wird,
der zweite Mikro-Strömungspfad (P2) einen mittleren Abschnitt des zweiten Mikro-Strömungspfads (P2b) aufweist, in dem das zweite modulare Rohmaterial (D2) angeordnet ist, und
die Querschnittsfläche des mittleren Abschnitts des zweiten Mikro-Strömungspfads (P2b), in dem das zweite Fluid strömt, sich ändert, wenn das zweite modulare Rohmaterial (D2) mittels des zweiten Fluids aufgelöst wird.

9. Der mikrofluidische Chip gemäß Anspruch 1, wobei der zweite Mikro-Strömungspfad (P2), der dritte Mikro-Strömungspfad (P3), der vierte Mikro-Strömungspfad (P4) und ein fünfter Mikro-Strömungspfad (P5) auf einer identischen Ebene gebildet sind, aber ein mittlerer Abschnitt des ersten Mikro-Strömungspfads (P1b) des ersten Mikro-Strömungspfads (P1), in dem das erste modulare Rohmaterial (D1) angeordnet ist, und ein mittlerer Abschnitt des zweiten Mikro-Strömungspfads (P2b) des zweiten Mikro-Strömungspfads (P2), in dem das zweite modulare Rohmaterial (D2) angeordnet ist, auf einer anderen Ebene als der zweite Mikro-Strömungspfad (P2) bis zum fünften Mikro-Strömungspfad (P5) gebildet sind.

10. Der mikrofluidische Chip gemäß Anspruch 1, wobei der erste Mikro-Strömungspfad (P1) bis zu einem fünften Mikro-Strömungspfad (P5) innerhalb einer ebenen Platte (T) gebildet sind und
die Platte (T) mit einem Vorsprungsabschnitt (T1) bereitgestellt ist, der zu dem ersten Mikro-Strömungspfad (P1) und dem zweiten Mikro-Strömungspfad (P2) hin vorsteht.

11. Eine Kosmetikherstellungsvorrichtung, die einen Aufbewahrungsabschnitt (100) aufweist, der konfiguriert ist, um ein erstes Fluid und ein zweites Fluid aufzubewahren,
einen mikrofluidischen Chip (400) gemäß Anspruch 1, der das erste Fluid und das zweite Fluid von dem Aufbewahrungsabschnitt (100) empfängt,
einen Hauptkörper (300), der konfiguriert ist, um den Aufbewahrungsabschnitt (100) und den mikrofluidischen Chip (400) aufzunehmen, und
wobei der mikrofluidische Chip (400) hinsichtlich des Hauptkörpers (300) austauschbar bereitgestellt ist.

## Revendications

1. Puce microfluidique, comprenant :
une partie d'entrée (410) comprenant une première entrée de fluide (411) à travers laquelle un premier fluide peut être introduit et une deuxième entrée de fluide (412) à travers laquelle un deuxième fluide peut être introduit ;
une partie de dissolution (420) comprenant un premier micro-chemin d'écoulement (P1) où une première matière première modulaire (D1) à dissoudre par le premier fluide est préchargée et un deuxième micro-chemin d'écoulement (P2) où une deuxième matière première modulaire (D2) à dissoudre par le deuxième fluide est préchargée ;
une confluence (430) comprenant un troisième micro-chemin d'écoulement (P3) où le premier fluide, la première matière première modulaire (D1) dissoute par le premier fluide, le deuxième fluide et la deuxième matière première modulaire (D2) dissoute par le deuxième fluide peuvent converger ;
une partie d'agitation (440) comprenant un quatrième micro-chemin d'écoulement (P4) qui est configuré pour former un fluide mélangé en mélangeant le premier fluide, la première matière première modulaire (D1) dissoute par le premier fluide, le deuxième fluide et la deuxième matière première modulaire (D2) dissoute par le deuxième fluide ; et
une partie de tourbillonnement (450) configurée pour former un contenu cosmétique comprenant des particules en créant un tourbillon et en coupant le fluide mélangé,
dans laquelle le quatrième micro-chemin d'écoulement (P4) prévu sur la partie d'agitation (440) comprend :
un premier chemin d'écoulement de mélange (4411) qui s'étend à partir du troisième micro-chemin d'écoulement (P3) formé sur la confluence (430) et s'étend dans une direction ;
un deuxième chemin d'écoulement de mélange (4412) ; et
un troisième chemin d'écoulement de mélange (4413),
dans laquelle au moins l'un parmi le premier chemin d'écoulement de mélange (4411) et le troisième chemin d'écoulement de mélange (4413) est formé selon une figure qui augmente puis diminue le long de la direction d'un écoulement de fluide,
**caractérisée en ce que**
le deuxième chemin d'écoulement de mélange (4412) est coudé selon un angle prédéfini par rapport au premier chemin d'écoulement de mélange (4411) et le troisième chemin d'écoulement de mélange (4413) est coudé selon un angle prédéfini par rapport au deuxième chemin d'écoulement (4412).

2. Puce microfluidique selon la revendication 1, dans laquelle le premier fluide introduit dans la première entrée de fluide (411) passe à travers le premier micro-chemin d'écoulement (P1) et dissout la première matière première modulaire (D1),
dans laquelle le deuxième fluide introduit dans la deuxième entrée de fluide (412) passe à travers le deuxième micro-chemin d'écoulement (P2) et dissout la deuxième matière première modulaire (D2).

3. Puce microfluidique selon la revendication 1, dans laquelle le premier fluide est fourni sous forme de fluide hydrophile,
dans laquelle le deuxième fluide est fourni sous forme de fluide hydrophobe,
dans laquelle la première matière première modulaire (D1) est formée dans une phase solide ou une phase gel qui peut être dissoute dans le fluide hydrophile, et
dans laquelle la deuxième matière première modulaire (D2) est formée dans une phase solide ou une phase gel qui peut être dissoute dans le fluide hydrophobe.

4. Puce microfluidique selon la revendication 1, dans laquelle un cinquième micro-chemin d'écoulement (P5) prévu sur la partie de tourbillonnement (450) comprend une pluralité de chemins d'écoulement formant des tourbillons (451) configurés pour créer un tourbillon en convertissant la direction de déplacement du fluide.

5. Puce microfluidique selon la revendication 4, dans laquelle chacun des chemins d'écoulement formant des tourbillons (451) comprend :
un premier chemin d'écoulement tournant (4511) qui dirige le fluide mélangé entrant pour qu'il tourne dans un sens ;
un deuxième chemin d'écoulement tournant (4512) qui dirige le fluide mélangé tournant dans un sens pour qu'il tourne dans l'autre sens ; et
un chemin d'écoulement de conversion de direction (4513) qui convertit le sens de rotation du fluide mélangé entre le premier chemin d'écoulement tournant (4511) et le deuxième chemin d'écoulement tournant (4512).

6. Puce microfluidique selon la revendication 4, dans laquelle la largeur du premier micro-chemin d'écoulement (P1) où la première matière première modulaire est disposée, ou la largeur du deuxième micro-chemin d'écoulement (P2) où la deuxième matière première modulaire est disposée, doit être formée plus grande que la largeur du cinquième micro-chemin d'écoulement (P2).

7. Puce microfluidique selon la revendication 1, dans laquelle les particules formées au niveau de la partie de tourbillonnement (450) sont des particules d'émulsion, et le contenu cosmétique comprenant les particules d'émulsion est fourni sous forme d'émulsion.

8. Puce microfluidique selon la revendication 1, dans laquelle le premier micro-chemin d'écoulement (P1) comprend une partie centrale du premier micro-chemin d'écoulement (P1b) où est disposée la première matière première modulaire (D1),
la surface de section transversale de la partie centrale du premier micro-chemin d'écoulement (P1b) où s'écoule le premier fluide change à mesure que la première matière première modulaire (D1) est dissoute par le premier fluide,
le deuxième micro-chemin d'écoulement (P2) comprend une partie centrale du deuxième micro-chemin d'écoulement (P2b) où la deuxième matière première modulaire (D2) est disposée, et
la surface de section transversale de la partie centrale du deuxième micro-chemin d'écoulement (P2b) où le deuxième fluide s'écoule change à mesure que la deuxième matière première modulaire (D2) est dissoute par le deuxième fluide.

9. Puce microfluidique selon la revendication 1, dans laquelle le deuxième micro-chemin d'écoulement (P2), le troisième micro-chemin d'écoulement (P3), le quatrième micro-chemin d'écoulement (P4) et un cinquième micro-chemin d'écoulement (P5) sont formés sur un plan identique, mais une partie centrale du premier micro-chemin d'écoulement (P1b) du premier micro-chemin (P1) où la première matière première modulaire (D1) est disposée et une partie centrale du deuxième micro-chemin d'écoulement (P2b) du deuxième micro-chemin d'écoulement (P2) où la deuxième matière première modulaire (D2) est disposée sont formées sur un plan différent de celui du deuxième micro-chemin d'écoulement (P2) au cinquième micro-chemin d'écoulement (P5).

10. Puce microfluidique selon la revendication 1, dans laquelle le premier micro-chemin d'écoulement (P1) à un cinquième micro-chemin d'écoulement (P5) sont formés à l'intérieur d'une plaque en forme plane (T), et
la plaque (T) est pourvue d'une partie en saillie (T1) qui fait saillie vers le premier micro-chemin d'écoulement (P1) et le deuxième micro-chemin d'écoulement (P2).

11. Appareil de fabrication de cosmétiques comprenant une partie de stockage (100) configurée pour stocker un premier fluide et un deuxième fluide ;
une puce microfluidique (400) selon la revendication 1 qui reçoit le premier fluide et le deuxième fluide provenant de la partie de stockage (100) ;
un corps principal (300) configuré pour loger la partie de stockage (100) et la puce microfluidique (400) ; et
la puce microfluidique (400) est fournie de manière remplaçable par rapport au corps principal (300).
